# EUROPEAN PATENT SPECIFICATION

(11) **EP 4 138 675 B1**
(45) Date of publication and mention of the grant of the patent: **30.07.2025**
(21) Application number: 21720242.3
(22) Date of filing: 19.04.2021
(51) Int. Cl.: A61B 10/00

(54) **STOOL SAMPLE DEVICE**
STUHLPROBENVORRICHTUNG
DISPOSITIF D'ÉCHANTILLON DE SELLES

(30) Priority: 21.04.2020 DK PA202070244
(43) Date of publication of application: 01.03.2023
(73) Proprietor: Region Sjælland, 4180 Sorø (DK)
(72) Inventor: BAANDRUP, Anders Ohlhues, 1820 Frederiksberg C (DK); KRAPPER, Anne-Dorte, 2640 Hedehusene (DK); HANSEN, Lasse Bremholm, 2605 Brøndby (DK)
(74) Representative: Zacco Denmark A/S
(86) International application number: PCT/EP2021/060081
(87) International publication number: WO 2021/213977

(56) References cited:
- WO-A1-2013/021274
- DE-A1- 102012 022 135
- JP-A- 2004 138 502
- JP-A- 2009 150 830
- JP-A- S6 465 451

## Description

### Field of the invention

The present invention relates to a stool sample device and a method for direct collection of an uncontaminated stool sample from the human rectum.

### Background of the invention

When a cancer patient in chemotherapy becomes hospitalised with acute diarrhea, it may be caused by several things, and in particular by 1) an infection with *Clostridium Difficile* (CD), 2) a chemotherapy-initiated diarrhea, or 3) other infectious diarrhea. This problem is large in all hospitals worldwide. CD is highly contagious, and in order to avoid spread of infection, it is therefore necessary to keep the patient in isolation, at least until the cause has been confirmed. While the patient is kept in isolation, a stool sample must be taken so that infection with either CD (or other types of contagious infectious induced diarrhea) or chemotherapy-initiated diarrhea can be confirmed, if one of these is the cause. This is the only way to have a confirmed diagnosis, and it is essential that the sample is not urine-contaminated or otherwise contaminated. As a result, the patients potentially end up being hospitalised up to three days due to stool samples that are urine-contaminated or otherwise contaminated, and the costs of hospitalisation for this reason are substantial. Accordingly, there is a need to find alternative ways of stool sampling and diagnosis in order to minimise the requirement for and the duration of hospitalisation due to diarrhea.

The above-mentioned scenario was the primary reason for the development of the present device, but the same field of problem applies for all isolated patients, where there is a need for uncontaminated stool samples, when they are hospitalised with a suspicion of potential, highly contagious gastrointestinal infections.

In addition, the device has a potential for use in other categories of hospitalised patients having suspected infections of contangious diarrhea, and who therefore need isolation; other potential area of use is in the primary care sector, where residents in eg. retirement facilities can have a quick and hygienic stool sample collected.

Chinese patent application CN2417832Y discloses a stool sample device for extracting a protected stool probe from a region of a human colon by insertion of the device into the colon, wherein the stool sampler comprises a cylindrical smooth body with a round head at the front end and a warning line, a toilet bowl, and a handle. After removal, the stool sample is protected from undesired contamination by being contained in the interior of the sample device until use and analysis. This method makes use of a scoop and prepares the sample for stool examination performed in the hospital.

JP 2004 138502 A discloses a stool sample device for collecting a stool sample from a region of a human rectum, comprising an outer part, and an inner part, having inner and outer units, comprising openings for collecting samples and having gaskets. The device makes use of annular grooves at the tip for sampling stool.

The main object of the present invention is to provide a new stool sample device that has improved function in comparison with the prior art sampler because it is easier to handle, more gentle to the patient, and requires shorter diagnosis time.

Another object is to provide a sample device that can be manufactured in cheap materials, and is easy to manufacture, and that has a high degree of reliability.

It is also an object to provide an improved and fast method for diagnosis of diarrhoea and its cause.

### Summary of the invention

The present invention relates to a stool sample device [A"] for collecting a stool sample from a region of a human rectum as defined in claim 1.

The present inventors have found that the invention can solve the problem of the requirement of prolonged hospitalisation. When a patient is admitted to the hospital with acute diarrhea expected to be infectious, and where there is a suspicion of CD or other contagious infectious induced cases of diarrhea, the hospital staff can collect a non-contaminated sample from a patient and analyse it substantially immediately to provide the sample test result within a very short time, such as within two or three hours. Accordingly, it does not take as much as three days to obtain a confirmation of the diagnosis as according to state of the art, and the hospital can easily, typically within three hours, find out if the patient will need to go into isolation or can be kept out of isolation.

The device of the invention can deliver a sample substantially immediately, and after sample analysis (two-three hours) the patient, if CD (or another diarrhea causing infection) was not the cause of diarrhea, can get out of hospital isolation. This quick analysis is more gentle to the patient than the method applied today, and at the same time it will save the hospital a lot of money.

The invention will also be applicable in other specialities and for screenings, and in a broader perspective, the device can be used in all clinical settings where patients in hospitals and residents in retirements facilities, within short notice, need to have stool samples taken that are not contaminated with urine or otherwise contaminated.

In addition, the device provides a significantly improvement in regards of hygiene and risk of contamination for the nursing staff who will be able to handle the potentially infectious stools in a much more confined area.

The present invention in another aspect relates to a method for collection of a uncontaminated stool sample from the human rectum as defined in claim 11.

Finally, the invention in a third aspect relates to a use of the stool sample device according to the invention for collecting a stool sample from a region of a human rectum.

### Drawing

Fig. 1a illustrates the stool sample device and its components.
Fig. 1b illustrates the device three-dimensionally.
Fig. 1c illustrates an example of applicable dimensions of a stool sample device of the invention.
Fig. 2a illustrates a specific embodiment of the stool sample device of the invention.
Fig. 2b illustrates an example of applicable dimensions for this embodiment of the stool sample device of the invention.
Fig. 3 illustrates another specific embodiment of the stool sample device of the invention.
Fig. 4 shows the device in function in a hospital test with standard laboratory pigs.

### Detailed description of the invention

In one embodiment of the invention in its three aspects, the inner unit(s) [E", E, E', F, F'] within the inner part of the stool sample device may be made up of a transparent material, such as a syntetic polymer, including polypropylene, e.g. nylon.

In a second embodiment of the invention in its three aspects, the gasket(s) [D", D, D'] may be made of a silicium containing polymer, such as medical rubber silicone.

In any of the above embodiments of the invention, the outer unit(s) [G", G, G', H, H'] within the outer part may be made up of a transparent material, such as a syntetic polymer, including polypropylene, e.g. nylon.

In any of the above embodiments of the invention, the material of the outer unit (G") or the outer tube [I, I'] may be flexible.

In any of the above embodiments of the invention, the material of the outer unit (G") or the outer tube [I, I'] may be transparent.

In any of the above embodiments of the invention, the outer unit [G", G, G'] may comprise 2, 3 or 4 openings for collecting sample, which openings may for example be square openings, such as elongated square openings, or round openings, such as circle- or oval-shaped openings. The openings are placed near to the tip end and assist to avoid excess pressure or vacuum during the stool sampling; in addition, the openings assist to avoid that the inner tube slides within the outer tube during the stool sampling.

In any of the above embodiments of the invention, the outer part may comprise a stop line for insertion of the device into the human rectum.

In one embodiment of the invention, a first outer unit [G'] and a second outer unit [H'] may be directly connected, and the second outer unit [H'], holding a second inner unit [F'], may have a cut thread in the opposite end relative to the handle [B'], and the first outer unit [G'] holding a first inner unit [E'] may hold a screw for attachment to the second outer unit [H'] in the cut thread. In this embodiment it is secured that the outer units [G'] and [H'] are hold firmly together when the device is removed from the human rectum.

In this embodiment of the invention, the second outer unit [G'] can be detached from the device at the attachment screw after extracting a stool probe and removing the device from the human rectum, thereby leaving the remaining part of the device holding the stool sample for sealing with a sealing [I'] at the opposite end relative to the handle [B', C'].

In this embodiment, the remaining part of the device holding the stool sample may have means for being sealed with a shipments screw that can be mounted at the cut thread in the second outer unit [H'] left after detachment of the second outer unit [G'].

These embodiments will allow for an easy sealing and handling of the stool probe that is to be send to analysis and diagnosis of the cause of diarrhoea.

The skilled person will know how to choose the dimensions of the device so that they fit the purpose.

In the method of the invention for collecting a stool sample, the handle [B", B, B'] is turned a sufficient number of times to collect the sample. Typically, the collection of a sample will require 4 to 10 turns, such as 4, 5, 6, 7, 8, 9, or 10 turns, e.g. 8 turns. Following this, the handle [C", C, C'] is pulled to a position wherein the inner unit [E", E, E'] has passed the openings in the outer unit [G", G, G'] so that no more stool is sampled, and the gasket(s) [D", D, D'] present in the inner unit(s) [E", E, E'] and [F, F'] have sealed the device to hold the stool probe with the outer tube [I, I'] or the outer unit [G", H, H'], and the device is removed from the region of the human rectum.

### Examples

### Example 1

One embodiment of the invention is now described as an example with reference to the drawing.

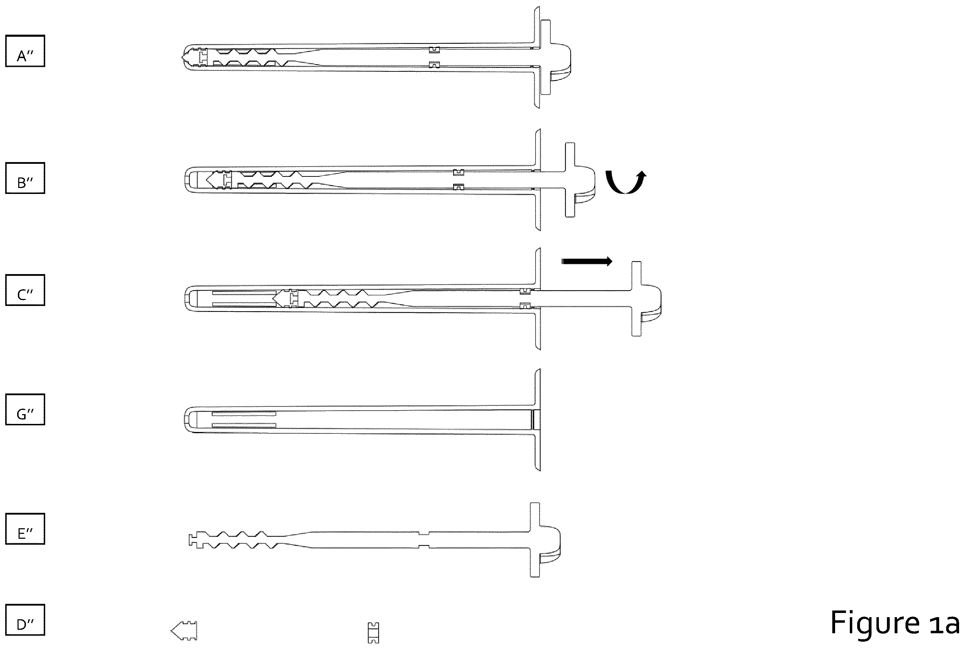

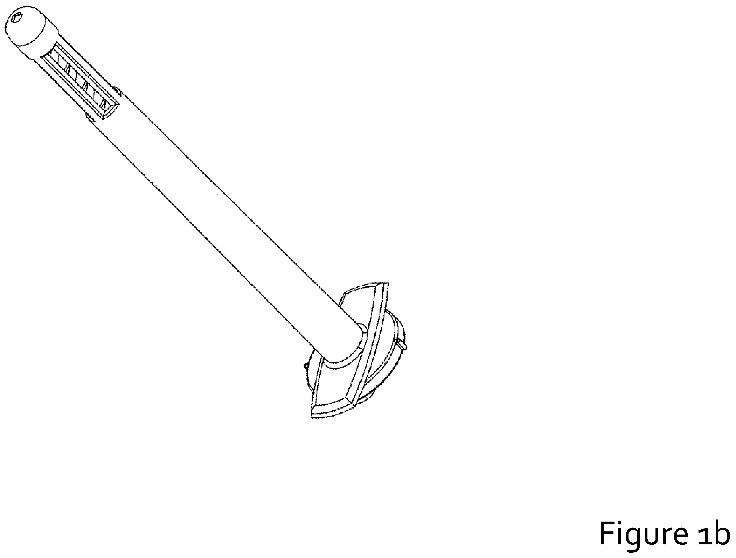

Figure 1a shows the stool sample device [A"] as the ready-for-use device and the stool sample device when in use, [B"] and [C"], and the following details:
[B"] When the device [A"] has been inserted into the human rectum in the right position, the person in charge of sampling the stool sample rotates the handle [B"] a sufficient number of times to collect sufficient sample material, whereby the outer unit [G"] holding the inner unit [E"] is filled with sample material.
[C"] The handle can then be pulled to place the inner unit [E"] in a position passed the openings in the outer part [G"], and the sample material will be kept within the inner part [E"] which is sealed with the silicone gaskets [D"] present in [E"].
[E"] is the inner unit having a spiral-shaped part or snail that collects the sample material. The device is provided with silicone gaskets [D"] that seales the unit [E"] in the tip end and the opposite end of the device when the handle is pulled.
[G"] is the outer unit wherein the inner unit [E"] holding the spiral-shaped part or snail is mounted. Sample material enters through the unit's four elongated square openings.

### Example 2

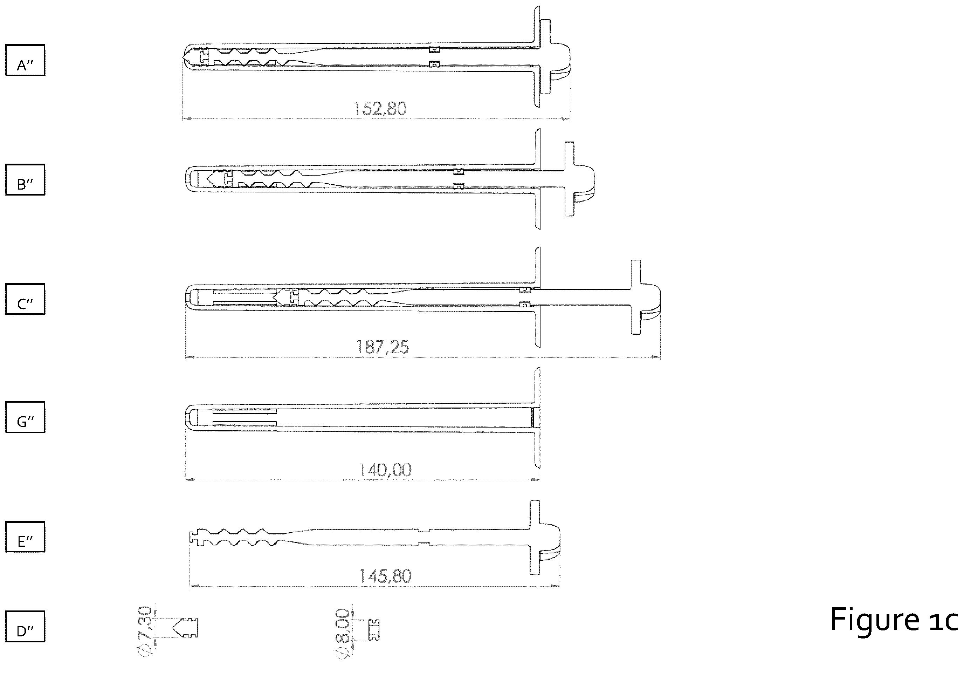

Figure 1c shows the same device and an example of applicable dimensions for the device.

In this embodiment of the device, the dimensions may be the following:
Length and diameter of the ready-for-use device: about 153 mm and about 8 mm;
Length of outer part G": about 140 mm;
Length and diameter of inner part E": about 146 mm.

### Example 3

Another embodiment of the invention is now described as an example with reference to the drawing.

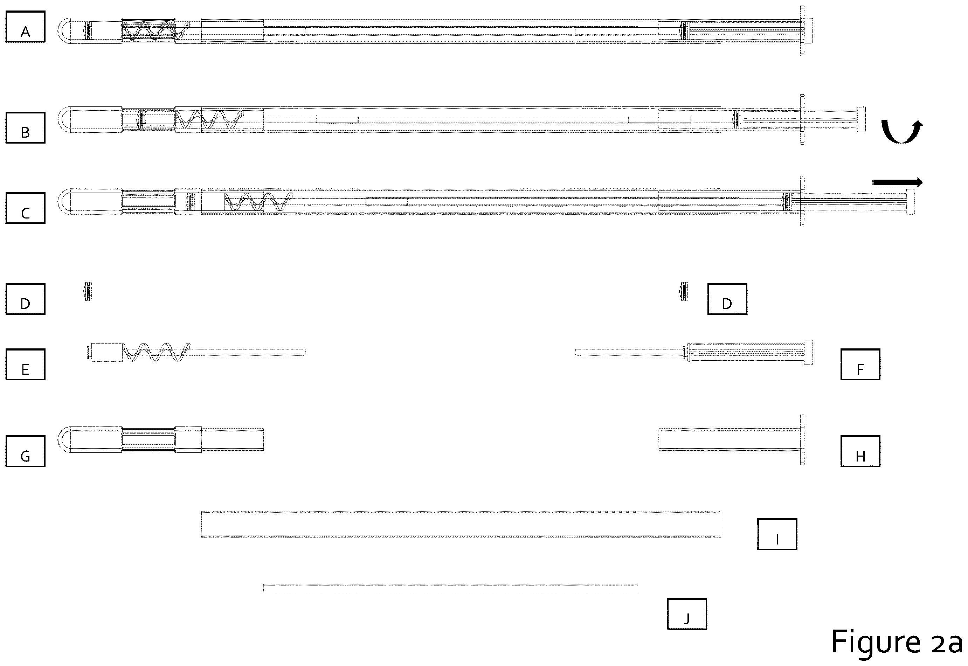

Figure 2a shows the stool sample device [A] as the ready-for-use device and the stool sample device when in use, [B] and [C], and the following details:
[B] When the device [A] has been inserted into the human rectum in the right position, the person in charge of sampling the stool sample rotates the handle [B] a sufficient number of times to collect sufficient sample material, whereby the outer unit [G] holding the inner unit [E] and the outer tube [I] are filled with sample material.
[C] The handle can then be pulled to place the inner unit [E] in a position passed the openings in the outer part [G], and the sample material will be kept within the inner part [E] and the outer tube [I] which is sealed with the silicone gaskets [D] present in [E] and [F].
[E] is the inner unit having a spiral-shaped part or snail that collects the sample material. The device is provided with a silicone gasket [D] that seales the unit [E] in the tip end of the device when the handle is pulled.
[F] is the part of the device having a handle that can be turned and pulled by the user. The device is provided with a silicone gasket [D] that partly produces vacuum when the handle is pulled and which seals the end of the outer tube [I] opposite to the outer unit [G].
[G] is the outer unit wherein the inner unit [E] holding the spiral-shaped part or snail is mounted. Sample material enters through the unit's four elongated square openings.
[H] is the outer unit wherein the inner unit with the handle is mounted.
[I] is the outer tube connecting the outer units [G] and [H].
[J] is the inner tube connecting the inner units [E] and [F].

### Example 4

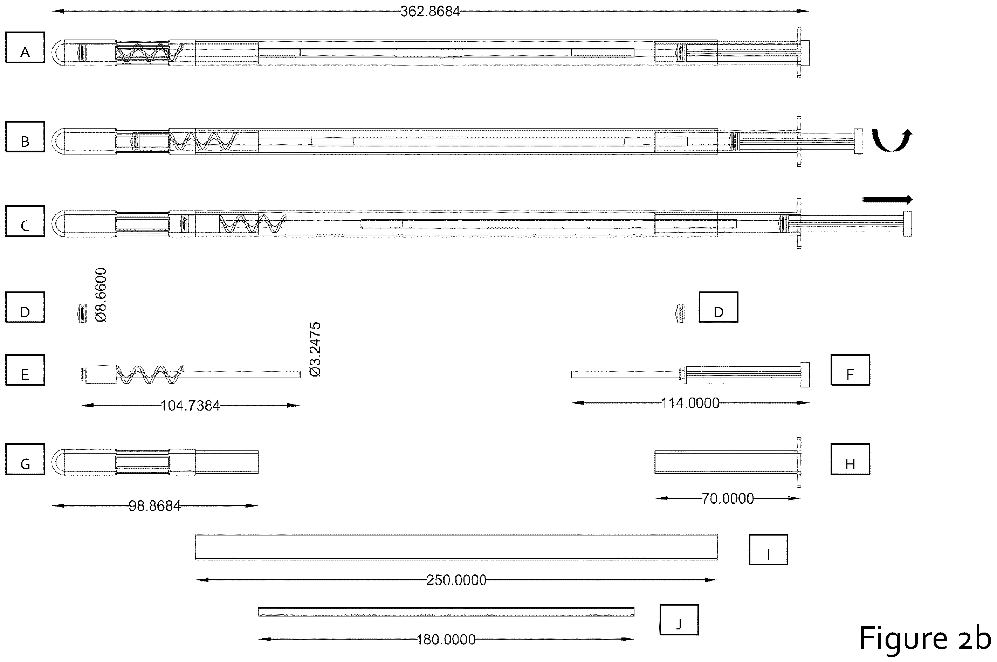

Figure 2b shows the same device and an example of applicable dimensions for the device.

In this embodiment of the device, the dimensions may be the following:
Length and diameter of the ready-for-use device: about 363 mm and about 12 mm;
Length of outer parts G, H and I: about 99 mm, about 70 mm, and about 250 mm, respectively;
Length and diameter of inner parts E, F, and J: about 105 mm, about 114 mm, and about 180 mm, respectively; diameter about 3.25 mm.

### Example 5

In one embodiment of the device the first outer unit [G'] holding the inner unit [E] can be detached from the device after extracting a stool probe and removing the device from the human rectum.

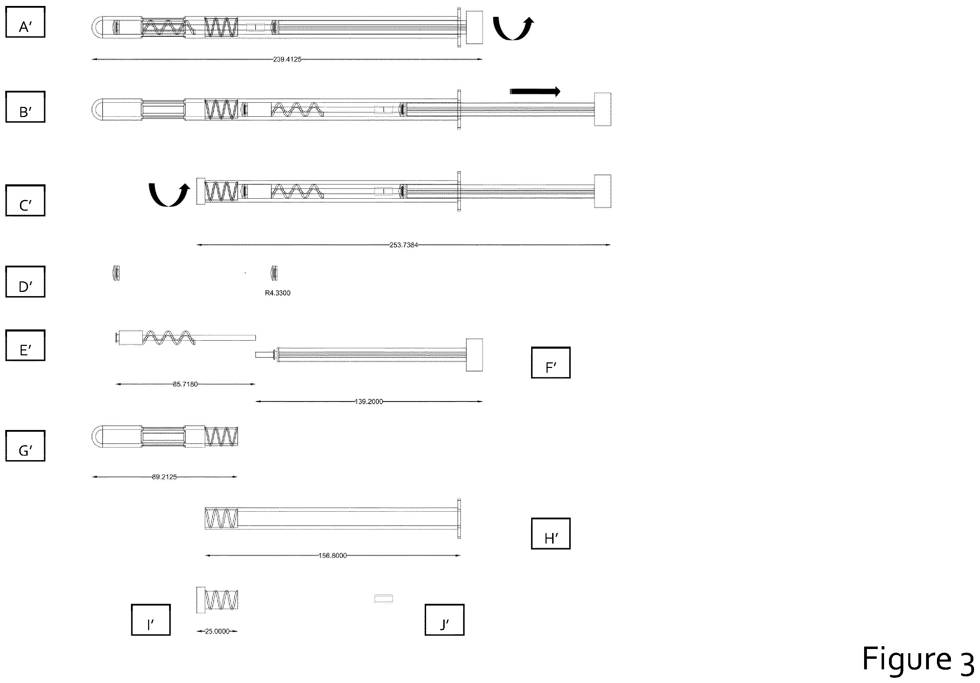

Figure 3 shows the stool sample device [A'] as the ready-for-use device and the stool sample device when in use, [B'] and [C'], and the following details; in this embodiment the outer parts [G'] and [H'] is directly connected by a cut thread and a screw (there is no outer tube):
[A'] When the device has been inserted into the human rectum in the right position, the person in charge of sampling the stool sample rotates the handle a sufficient number of times to collect sufficient sample material, whereby the outer units [G'] and [H'] are filled with sample material.
[B'] The handle can then be pulled to place the inner unit [E'] in a position passed the openings in the outer part [G'], and the sample material will be kept within the inner part [E'] and the outer part [H'] which are sealed with the silicone gaskets [D'] present in [E'] and [F'].
[C'] The outer part [G'] can be removed and the end of the outer part [H'] is mounted with a shipments screw [I'].
[E'] is the inner unit having a spiral-shaped part or snail that collects the sample material. The device is provided with a silicone gasket [D'] that seales the unit [E'] in the tip end of the device when the handle is pulled.
[F'] is the part of the device having a handle that can be turned and pulled by the user. The device is provided with a silicone gasket [D'] that partly produces vacuum when the handle is pulled and which seals the end of the outer tube [H'] opposite to the outer unit [G'].
[G'] is the outer unit wherein the inner unit [E'] holding the spiral-shaped part is mounted. Sample material enters through the unit's four elongated square openings.
[H'] is the outer unit wherein the inner unit [F'] with the handle is mounted.
[J'] is the inner tube connecting the inner units [E'] and [F'].

### Example 6

A prototype of the device has in been tested on pigs in a controlled laboratory setting, under strict veterinarian control, and with the test animals in general anestesia. In this test the device proved functional, easily used, and performing in accordance to the theoretic expectations of the inventors.

### Test 1 of rectal stool sample device

### Conditions

*Test site*: University Hospital of Southern Denmark animal stables, operating theatre.

*Test subjects*: 2 standard laboratory pigs in general anesthesia, prepared and sedated for surgical training.

*Test aim*: Evaluation of prototype in relation to anatomy, user conditions, acquiring effect and safety in regards to mucosa damage and rectal lesions.

### Description:

The test proto type was lubricated with standard rectal lubrication gel, the device was inserted in the rectum, and the "snail" was rotated clockwise, and retracted. Afterwards the rectum was inspected with a recto scope (devise for visual inspection). The procedure was documented by video and pictures. The procedure was completed with both test animals.

### Results

The device was easily and without resistance inserted, the "snail" was easily rotated. There was no faeces in the rectum, but the device obtained rectal mucus. The device was easily removed from the rectum. Subsequent inspection showed no harm to the rectal mucosa (the inside of the rectum), and there was no blood on the device indicating any rectal lesions.

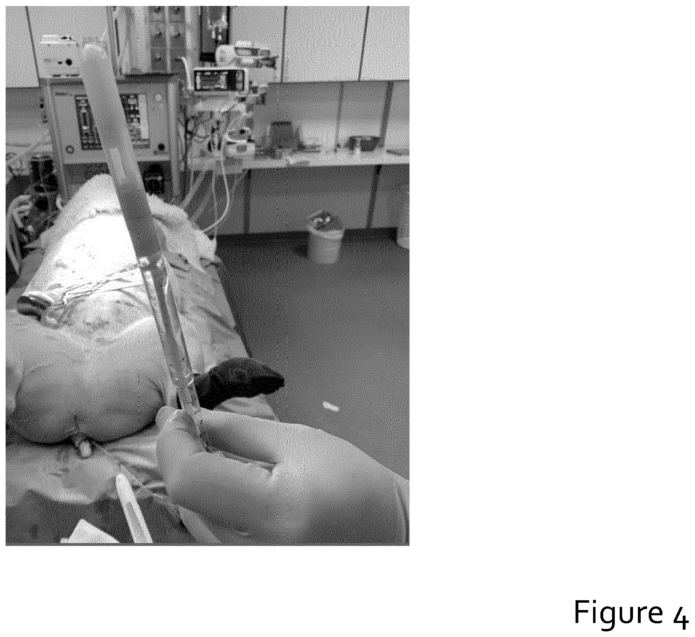

Figure 4 shows the test device with mucus.

### Example 7

### Test 2 of rectal stool sample device

### Conditions

*Test site:* Zealand University Hospital
*Test mannequin*: Artificial human colon and rectum, in plastic and rubber, primarily designed to train in endoscopy.

*Test aim*: Evaluation of prototype in relation to acquiring effect.

### Description:

The test prototype was lubricated with standard rectal lubrication gel, the device was inserted in the rubber rectum, and the "snail" was rotated clockwise, and retracted. A scope was inserted from the colon, and thereby was able to film the device in the artificial rectum. The rectum was filled with a liquid substance mimicking stool in the consistence of diarrhea. Video and pictures documented the procedure.

### Results

The procedure was repeated 10 times, and each time sufficient material was acquired.

## Claims

1. Stool sample device [A"] for collecting a stool sample from a region of a human rectum, which device comprises an outer part and an inner part, wherein
the outer part comprises an outer unit [G"], wherein an inner unit [E"] is mounted, further wherein the outer unit [G"] has a round tip and comprises openings for collecting sample from the human rectum; and
the inner part comprises the inner unit [E"] fitted with gaskets [D"], a first gasket [D"] is placed at the tip end of the inner unit [E"], and a second gasket is placed at the opposite end of a spiral-shaped part or snail, and at distance of a handle [B"];
**characterised in that**
the inner unit [E"] is equipped with the spiral-shaped part or snail placed at the tip end relative to the outer unit [G"] and with the handle for turning [B"] and pulling [C"] at the opposite end relative to the spiral-shaped part or snail; and
that the inner unit [E"] can be turned and pulled back by the handle [B", C"] with respect to the outer unit [G''].

2. Stool sample device [A] according to claim 1, **characterised in that**
the outer part is two-parted or three-parted and comprises a first outer unit [G] and a second outer unit [H], each holding one of two separate inner units [E, F] of the inner part, and further wherein the two outer units [G, H] are either directly connected or are separated and connected by a cylindrical outer tube [I] surrounding an inner tube [J]; and
the inner part is an assembly of multiple units and comprises the two separate inner units [E, F] each fitted with a gasket [D]; wherein
the first inner unit [E] is mounted within the first outer unit [G] and is equipped with the spiral-shaped part or snail placed at the tip end relative to the outer unit [G], and wherein the second inner unit [F] is mounted within the second outer unit [H] and is equipped with the handle for turning [B] and pulling [C], further wherein the two separate inner units [E, F] are connected by a cylindrical inner tube [J], the gasket [D] of the first inner unit [E] is placed at the tip end relative to the outer unit [G], and the gasket [D] of the second inner unit [F] is placed at the opposite end relative to the handle [B, C]; and wherein
the inner part consisting of the inner units [E, F] and the inner tube [J] can be turned and pulled back by the handle [B, C] with respect to the outer unit [G].

3. The stool sample device according to any of the claims 1 and 2, wherein the inner unit(s) [E", E, F] within the inner part, and/or the outer unit(s) [G", G, H] within the outer part, are made up of a transparent material, such as a syntetic polymer, including polypropylene, e.g. nylon.

4. The stool sample device according to any of the claims 1 to 3, wherein the gasket(s) [D", D] is made of a silicium containing polymer, such as medical rubber silicone.

5. The stool sample device according to any of the claims 1 to 4, wherein the material of the outer part [G"] or the outer tube [I] is flexible.

6. The stool sample device according to any of the claims 1 to 5, wherein the material of the outer part [G"] or the outer tube [I] is transparent.

7. The stool sample device according to any of the claims 1 to 6, wherein the outer unit [G", G] comprises 2, 3, or 4 openings for collecting sample, which openings may be square openings, such as elongated square openings, or round openings, such as circle- or oval-shaped openings.

8. The stool sample device according to any of the claims 2 to 7, wherein the first outer unit [G'] and the second outer unit [H'] are directly connected, wherein the second outer unit [H'], holding the second inner unit [F'], has a cut thread in the opposite end relative to the handle [B'], and wherein the first outer unit [G'] holding the first inner unit [E'] holds a screw for attachment to the second outer unit [H'] in the cut thread.

9. The stool sample device according to any of the claims 2 to 8, wherein the first outer unit [G'] is detachable from the device at the attachment screw after extraction of a stool probe and removal of the device from the human rectum, thereby leaving the remaining part of the device holding the stool sample for sealing with a sealing [I'] at the opposite end relative to the handle [B', C'].

10. The stool sample device according to claim 9, wherein the remaining part of the device holding the stool sample has means for being sealed with a shipments screw [I'] that can be mounted at the cut thread in the second outer unit [H'] left after detachment of the first outer unit [G'].

11. Method for collection of a stool sample from the human rectum, **characterised in that** the stool sample device according to any of the claims 1-10 is inserted into the human rectum, that the handle [B", B, B'] of the device is turned sufficiently for collecting an uncontaminated sample, whereafter the handle [C", C, C'] is pulled to a position wherein the inner unit [E", E, E'] has passed the openings in the outer unit [G", G, G'], and the gaskets [D", D, D'] present in the inner units [E", E, E'] and [F, F'] have sealed the device to hold the stool sample within the outer tube [I] or the outer unit [G", H'], and the device is removed from the region of the human rectum.

12. Method according to claim 11, wherein the handle is turned 4 to 10 times, such as 4, 5, 6, 7, 8, 9, or 10 times, e.g. 8 times.

13. Use of the stool sample device according to any of the claims 1 to 10 for collecting a stool sample from a region of a human rectum.

## Patentansprüche

1. Stuhlprobenvorrichtung [A"] zum Entnehmen einer Stuhlprobe aus einem Bereich eines menschlichen Rektums, wobei die Vorrichtung ein äußeres Teil und ein inneres Teil umfasst, wobei das äußere Teil eine äußere Einheit [G"] umfasst, wobei eine innere Einheit [E"] montiert ist, ferner wobei die äußere Einheit [G"] eine runde Spitze aufweist und Öffnungen zum Sammeln einer Probe aus dem menschlichen Rektum umfasst; und das innere Teil die innere Einheit [E"] umfasst, die mit Dichtungen [D"] ausgerüstet ist, wobei eine erste Dichtung [D"] an dem spitzen Ende der inneren Einheit [E"] platziert ist und eine zweite Dichtung an dem gegenüberliegenden Ende eines spiralförmigen Teils oder einer Schnecke und in einem Abstand zu einem Griff [B"] platziert ist;
**dadurch gekennzeichnet, dass**
die innere Einheit [E"] mit dem spiralförmigen Teil oder der Schnecke, das/die relativ zu der äußeren Einheit [G"] an dem spitzen Ende und mit dem Griff zum Drehen [B"] und Ziehen [C"] an dem gegenüberliegenden Ende relativ zu dem spiralförmigen Teil oder der Schnecke platziert ist, ausgestattet ist; und
dass die innere Einheit [E"] durch den Griff [B", C"] gegenüber der äußeren Einheit [G"] gedreht und zurückgezogen werden kann.

2. Stuhlprobenvorrichtung [A] nach Anspruch 1, **dadurch gekennzeichnet, dass** das äußere Teil zweiteilig oder dreiteilig ist und eine erste äußere Einheit [G] und eine zweite äußere Einheit [H] umfasst, wobei jede eine von zwei separaten inneren Einheiten [E, F] des inneren Teils hält und ferner wobei die beiden äußeren Einheiten [G, H] entweder direkt verbunden sind oder getrennt und durch ein zylindrisches äußeres Rohr [I] verbunden sind, das ein inneres Rohr [J] umgibt; und
das innere Teil eine Baugruppe aus mehreren Einheiten ist und die zwei separaten inneren Einheiten [E, F] umfasst, die jeweils mit einer Dichtung [D] ausgerüstet sind; wobei
die erste innere Einheit [E] innerhalb der ersten äußeren Einheit [G] montiert ist und mit dem spiralförmigen Teil oder der Schnecke ausgestattet ist, die an dem spitzen Ende relativ zu der äußeren Einheit [G] platziert ist, und wobei die zweite innere Einheit [F] innerhalb der zweiten äußeren Einheit [H] montiert ist und mit dem Griff zum Drehen [B] und Ziehen [C] ausgestattet ist, ferner wobei die zwei separaten inneren Einheiten [E, F] durch ein zylindrisches inneres Rohr [J] verbunden sind, die Dichtung [D] der ersten inneren Einheit [E] relativ zu der äußeren Einheit [G] an dem spitzen Ende angeordnet ist und die Dichtung [D] der zweiten inneren Einheit [F] relativ zu dem Griff [B, C] an dem gegenüberliegenden Ende angeordnet ist; und wobei
das aus den inneren Einheiten [E, F] und dem inneren Rohr [J] bestehende innere Teil durch den Griff [B, C] gegenüber der äußeren Einheit [G] gedreht und zurückgezogen werden kann.

3. Stuhlprobenvorrichtung nach einem der Ansprüche 1 und 2, wobei die innere(n) Einheit(en) [E", E, F] innerhalb des inneren Teils und/oder die äußere(n) Einheit(en) [G", G, H] innerhalb des äußeren Teils aus einem transparenten Material, wie einem synthetischen Polymer, einschließlich Polypropylen, z. B. Nylon, bestehen.

4. Stuhlprobenvorrichtung nach einem der Ansprüche 1 bis 3, wobei die Dichtung(en) [D", D] aus einem siliziumhaltigen Polymer, wie beispielsweise medizinischem Kautschuksilikon, hergestellt sind.

5. Stuhlprobenvorrichtung nach einem der Ansprüche 1 bis 4, wobei das Material des äußeren Teils [G"] oder des äußeres Rohrs [I] flexibel ist.

6. Stuhlprobenvorrichtung nach einem der Ansprüche 1 bis 5, wobei das Material des äußeren Teils [G"] oder des äußeres Rohrs [I] transparent ist.

7. Stuhlprobenvorrichtung nach einem der Ansprüche 1 bis 6, wobei die äußere Einheit [G", G] 2, 3 oder 4 Öffnungen zum Sammeln von Proben umfasst, wobei es sich um quadratische Öffnungen, wie längliche quadratische Öffnungen, oder um runde Öffnungen, wie kreis- oder ovalförmige Öffnungen, handeln kann.

8. Stuhlprobenvorrichtung nach einem der Ansprüche 2 bis 7, wobei die erste äußere Einheit [G'] und die zweite äußere Einheit [H'] direkt verbunden sind, wobei die zweite äußere Einheit [H'], die die zweite innere Einheit [F'] hält, an dem gegenüberliegenden Ende relativ zu dem Griff [B'] ein geschnittenes Gewinde aufweist und wobei die erste äußere Einheit [G'], die die erste innere Einheit [E'] hält, eine Schraube zur Befestigung an der zweiten äußeren Einheit [H'] in dem geschnittenen Gewinde hält.

9. Stuhlprobenvorrichtung nach einem der Ansprüche 2 bis 8, wobei die erste äußere Einheit [G'] nach der Entnahme einer Stuhlprobe und der Entfernung der Vorrichtung aus dem menschlichen Rektum an der Befestigungsschraube von der Vorrichtung abnehmbar ist, wodurch das verbleibende Teil der Vorrichtung, das die Stuhlprobe hält, zum Abdichten mit einer Dichtung [I'] an dem gegenüberliegenden Ende relativ zu dem Griff [B', C'] bleibt.

10. Stuhlprobenvorrichtung nach Anspruch 9, wobei das verbleibende Teil der Vorrichtung, das die Stuhlprobe hält, Mittel zum Abdichten mit einer Versandschraube [I'] aufweist, die an dem geschnittenen Gewinde in der zweiten äußeren Einheit [H'] angebracht werden kann, die nach dem Lösen der ersten äußeren Einheit [G'] übrig bleibt.

11. Verfahren zur Entnahme einer Stuhlprobe aus dem menschlichen Rektum, **dadurch gekennzeichnet, dass** die Stuhlprobenvorrichtung nach einem der Ansprüche 1-10 in das menschliche Rektum eingeführt wird, dass der Griff [B", B, B'] der Vorrichtung ausreichend gedreht ist, um eine nicht kontaminierte Probe zu entnehmen, woraufhin der Griff [C", C, C'] in eine Position gezogen wird, in der die innere Einheit [E", E, E'] die Öffnungen in der äußeren Einheit [G", G, G'] passiert hat und die in den inneren Einheiten [E", E, E'] und [F, F'] vorhandenen Dichtungen [D", D, D'] die Vorrichtung zum Halten der Stuhlprobe innerhalb des äußeren Rohrs [I] oder der äußeren Einheit [G", H'] abgedichtet haben, und die Vorrichtung aus dem Bereich des menschlichen Rektums entfernt wird.

12. Verfahren nach Anspruch 11, wobei der Griff 4- bis 10-mal gedreht wird, wie etwa 4-, 5-, 6-, 7-, 8-, 9- oder 10-mal, z. B. 8-mal.

13. Verwendung der Stuhlprobenvorrichtung nach einem der Ansprüche 1 bis 10 zum Entnehmen einer Stuhlprobe aus einem Bereich eines menschlichen Rektums.

## Revendications

1. Dispositif d'échantillon de selles [A"] pour la collecte d'échantillons de selles dans une région d'un rectum humain, lequel dispositif comprend une partie externe et une partie interne, dans lequel
la partie externe comprend une unité externe [G"], dans laquelle une unité interne [E"] est montée, en outre dans lequel l'unité externe [G"] a une pointe ronde et comprend des ouvertures pour la collecte d'échantillon depuis le rectum humain ; et
la partie interne comprend l'unité interne [E"] équipée de joints [D"], un premier joint [D"] est placé à l'extrémité de point de l'unité interne [E"], et un deuxième joint est placé à l'extrémité opposée d'une partie en forme de spirale ou d'escargot, et à distance d'une poignée [B"] ;
**caractérisé en ce que**
l'unité interne [E"] est équipée de la partie en forme de spirale ou d'escargot placée à l'extrémité de pointe par rapport à l'unité externe [G"] et de la poignée pour tourner [B"] et tirer [C"] à l'extrémité opposée par rapport à la partie en forme de spirale ou d'escargot ; et
que l'unité interne [E"] peut être tournée et tirée vers l'arrière par la poignée [B", C"] par rapport à l'unité externe [G"].

2. Dispositif d'échantillon de selles [A] selon la revendication 1, **caractérisé en ce que** la partie externe est en deux ou trois parties et comprend une première unité externe [G] et une deuxième unité externe [H], chacune contenant l'une des deux unités internes distinctes [E, F] de la partie interne, et en outre dans lequel les deux unités externes [G, H] sont soit directement connectées, soit séparées et connectées par un tube externe cylindrique [I] entourant un tube interne [J] ; et
la partie interne est un assemblage de multiples unités et comprend les deux unités internes séparées [E, F] chacune équipée d'un joint [D] ; dans lequel
la première unité interne [E] est montée à l'intérieur de la première unité externe [G] et est équipée de la partie en forme de spirale ou d'escargot placé à l'extrémité de pointe par rapport à l'unité externe [G], et dans lequel la deuxième unité interne [F] est montée à l'intérieur de la deuxième unité externe [H] et est équipée d'une poignée pour tourner [B] et tirer [C], en outre dans lequel les deux unités internes séparées [E, F] sont reliées par un tube interne cylindrique [J], le joint [D] de la première unité interne [E] est placé à l'extrémité de pointe par rapport à l'unité externe [G] et le joint [D] de la deuxième unité interne [F] est placé à l'extrémité opposée par rapport à la poignée [B, C] ; et dans lequel
la partie interne constituée des unités internes [E, F] et du tube interne [J] peut être tournée et tirée vers l'arrière par la poignée [B, C] par rapport à l'unité externe [G].

3. Dispositif d'échantillon de selles selon l'une quelconque des revendications 1 et 2, dans lequel la ou les unités internes [E", E, F] à l'intérieur de la partie interne, et/ou la ou les unités externes [G", G, H] à l'intérieur de la partie externe, sont constituées d'un matériau transparent, tel qu'un polymère synthétique, comprenant du polypropylène, par exemple du nylon.

4. Dispositif d'échantillon de selles selon l'une quelconque des revendications 1 à 3, dans lequel le ou les joints [D", D] sont constitués d'un polymère contenant du silicium, tel que du caoutchouc de silicone médical.

5. Dispositif d'échantillon de selles selon l'une quelconque des revendications 1 à 4, dans lequel le matériau de la partie externe [G"] ou du tube externe [I] est flexible.

6. Dispositif d'échantillon de selles selon l'une quelconque des revendications 1 à 5, dans lequel le matériau de la partie externe [G"] ou du tube externe [I] est transparent.

7. Dispositif d'échantillon de selles selon l'une quelconque des revendications 1 à 6, dans lequel l'unité externe [G", G] comprend 2, 3 ou 4 ouvertures pour collecter l'échantillon, lesquelles ouvertures peuvent être des ouvertures carrées, telles que des ouvertures carrées allongées, ou des ouvertures rondes, telles que des ouvertures de forme circulaire ou ovale.

8. Dispositif d'échantillon de selles selon l'une quelconque des revendications 2 à 7, dans lequel la première unité externe [G'] et la deuxième unité externe [H'] sont directement reliées, dans lequel la deuxième unité externe [H'], portant la deuxième unité interne [F'], a un filetage coupé dans l'extrémité opposée par rapport à la poignée [B'], et dans lequel la première unité externe [G'] portant la première unité interne [E'] porte une vis pour la fixation à la deuxième unité externe [H'] dans le filetage coupé.

9. Dispositif d'échantillon de selles selon l'une quelconque des revendications 2 à 8, dans lequel la première unité externe [G'] est détachable du dispositif au niveau de la vis de fixation après l'extraction d'une sonde de selles et le retrait du dispositif du rectum humain, laissant ainsi la partie restante du dispositif contenant l'échantillon de selles pour le scellement avec un joint [I'] à l'extrémité opposée par rapport à la poignée [B', C'].

10. Dispositif d'échantillon de selles selon la revendication 9, dans lequel la partie restante du dispositif contenant l'échantillon de selles comporte des moyens pour être scellée avec une vis d'expédition [I'] qui peut être montée au niveau du filetage coupé dans la deuxième unité externe [H'] laissée après le détachement de la première unité externe [G'].

11. Procédé de collecte d'un échantillon de selles à partir du rectum humain, **caractérisé en ce que** le dispositif d'échantillon de selles selon l'une quelconque des revendications 1 à 10 est inséré dans le rectum humain, que la poignée [B", B, B'] du dispositif est tournée suffisamment pour collecter un échantillon non contaminé, après quoi la poignée [C", C, C'] est tirée jusqu'à une position dans laquelle l'unité interne [E", E, E'] a dépassé les ouvertures de l'unité externe [G", G, G'], et les joints [D", D, D'] présents dans les unités internes [E", E, E'] et [F, F'] ont scellé le dispositif pour maintenir l'échantillon de selles à l'intérieur du tube externe [I] ou de l'unité externe [G", H'], et le dispositif est retiré de la région du rectum humain.

12. Procédé selon la revendication 11, dans lequel la poignée est tournée 4 à 10 fois, par exemple 4, 5, 6, 7, 8, 9 ou 10 fois, par exemple 8 fois.

13. Utilisation du dispositif d'échantillon de selles selon l'une quelconque des revendications 1 à 10 pour collecter un échantillon de selles à partir d'une région d'un rectum humain.
